# EUROPEAN PATENT APPLICATION

(11) **EP 4 811 393 A2**
(43) Date of publication of application: **23.09.2026**
(21) Application number: 26190564.0
(22) Date of filing: 29.12.2021
(51) Int. Cl.: G16H 50/30

(54) **MONITORING SYSTEM FOR PRESSURE INJURY**

(30) Priority: 30.12.2020 US 202063131851 P; 21.12.2021 US 202117557554
(62) Document number(s) of the earlier application(s) in accordance with Art. 76 EPC: 21218124.2 / 4 024 409
(71) Applicant: Hill-Rom Services, Inc., Batesville, IN 47006-9167 (US)
(72) Inventor: KAYSER, Susan, Batesville, 47006 (US); Le GALL, Marion, Batesville, 47006 (US); ZHOU, Jie, Batesville, 47006 (US); PFEFFER, Mary L, Batesville, 47006 (US); RIZZO, Jennifer M, Batesville, 47006 (US)
(74) Representative: Reddie & Grose LLP

(57) **Abstract**

A method of tracking pressure injuries, comprising: collecting data from an electronic medical record of a patient; collecting data from a support apparatus relating to use of the support apparatus; calculating a risk score of developing a pressure injury in response to the data received from at least one of the electronic medical record and the support apparatus; generating a notification including at least one of the risk score, a listing of risk factors, and areas of increased risk; and generating treatment orders based on at least one of the risk score, the listing of risk factors, and the areas of increased risk.

## Description

The present disclosure generally relates to a monitoring system, and more particularly to a monitoring system for a pressure injury.

The present invention is defined in the appended claims.

According to one aspect of the present disclosure, a method of tracking pressure injuries, comprises: collecting data from an electronic medical record of a patient; collecting data from a support apparatus relating to use of the support apparatus; calculating a risk score of developing a pressure injury in response to the data received from at least one of the electronic medical record and the support apparatus; generating a notification including at least one of the risk score, a listing of risk factors, and areas of increased risk; and generating treatment orders based on at least one of the risk score, the listing of risk factors, and the areas of increased risk.

According to one aspect of the present disclosure, a monitoring system for tracking pressure related injury risk includes a support apparatus with a controller and communication circuitry configured to communicate via a communication network. A control unit is configured to communicate with at least one of a remote server and the support apparatus via the communication network. The control unit is configured to determine at least one of a risk status and a risk score for developing a pressure injury in response to communication from the at least one of the remote server and the support apparatus, determine at least one contributing risk factor for developing the pressure injury, and generate a notification configured to be communicated to a user interface assembly.

According to another aspect of the present disclosure, a monitoring system for tracking pressure related injury risk includes a support apparatus with a controller. The controller is configured to communicate data relating to the support apparatus. Communication circuitry is operably coupled to the support apparatus. The communication circuitry is configured to communicate the data via a communication network. A control unit is configured to communicate with the support apparatus and a user interface assembly via the communication network. The control unit is configured to determine at least one of a risk status and a risk score for developing a pressure injury, determine an area of increased risk for developing the pressure injury; and generate a notification including a graphical representation of a person that is displayed via the user interface assembly.

According to yet another aspect of the present disclosure, a monitoring system for tracking pressure related injury risk includes a treatment device with a controller. The controller is configured to communicate data relating to the treatment device. Communication circuitry is operably coupled to the treatment device. The communication circuitry is configured to communicate the data via a communication network. A control unit that communicates with at least one of the treatment device and a user interface assembly via the communication network. The control unit is configured to determine at least one of a risk status and a risk score for developing a pressure injury and generate a first notification configured to be communicated to the user interface assembly. The first notification includes a selectable feature. The selectable feature corresponds to at least one of a risk status indicator, a risk score indicator, a risk assessment, a risk area, and a prevention treatment order. The control unit is configured to generate a second notification in response to a selection of at least one of the selectable features. The control unit is configured to communicate the second notification to the user interface assembly.

The invention will now be further described by way of example with reference to the accompanying drawings, in which:
FIG. 1 is a schematic diagram of a portion of a medical facility, according to the present disclosure;
FIG. 2 is a block diagram of a monitoring system with various devices communicating via a communication interface, according to the present disclosure;
FIG. 3 is a schematic diagram of a portion of a room environment within a medical facility with various treatment devices, according to the present disclosure;
FIG. 4 is a schematic diagram of a portion of a surgical suite within a medical facility with various treatment devices, according to the present disclosure;
FIG. 5 is a block diagram of a monitoring system with various devices communicating via a communication interface, according to the present disclosure;
FIG. 6 is a block diagram of a support apparatus wirelessly communicating with a local server via a wireless access transceiver, according to the present disclosure;
FIG. 7 is a block diagram of treatment devices wirelessly communicating with a local server via wireless access points, according to the present disclosure;
FIG. 8 is representative of an application interface providing a communication portal, according to the present disclosure;
FIG. 9 is representative of an application interface providing a patient profile, according to the present disclosure;
FIG. 10 is representative of an application interface providing risk information, according to the present disclosure;
FIG. 11 is representative of an application interface providing a graphical illustration of patient risk areas, according to the present disclosure;
FIG. 12 is representative of an application interface providing treatment orders, according to the present disclosure;
FIG. 13 is representative of an application interface providing a discharge report, according to the present disclosure;
FIG. 14 is representative of an application interface providing a feedback report, according to the present disclosure;
FIG. 15 is a flow diagram of a method of monitoring and tracking risk of an intraoperative pressure injury, according to the present disclosure;
FIG. 16 is a flow diagram of a method of monitoring and tracking risk of an intraoperative pressure injury, according to the present disclosure;
FIG. 17 is a flow diagram of a method of monitoring and tracking risk of a medical device related pressure injury, according to the present disclosure; and
FIG. 18 is a flow diagram of a method of monitoring and tracking risk of a medical device related pressure injury, according to the present disclosure.

The present illustrated embodiments reside primarily in combinations of method steps and apparatus components related to a monitoring system for a pressure injury. Accordingly, the apparatus components and method steps have been represented, where appropriate, by conventional symbols in the drawings, showing only those specific details that are pertinent to understanding the embodiments of the present disclosure so as not to obscure the disclosure with details that will be readily apparent to those of ordinary skill in the art having the benefit of the description herein. Further, like numerals in the description and drawings represent like elements.

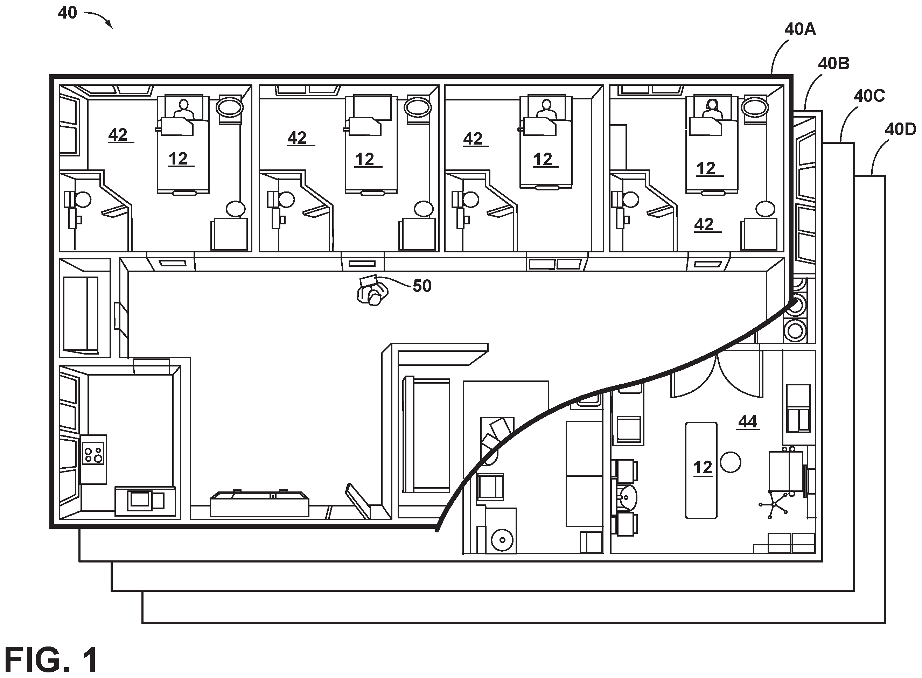

For purposes of description herein, the terms "upper," "lower," "right," "left," "rear," "front," "vertical," "horizontal," and derivatives thereof, shall relate to the disclosure as oriented in FIG. 1. Unless stated otherwise, the term "front" shall refer to a surface closest to an intended viewer, and the term "rear" shall refer to a surface furthest from the intended viewer. However, it is to be understood that the disclosure may assume various alternative orientations, except where expressly specified to the contrary. It is also to be understood that the specific structures and processes illustrated in the attached drawings, and described in the following specification are simply exemplary embodiments of the inventive concepts defined in the appended claims. Hence, specific dimensions and other physical characteristics relating to the embodiments.

The terms "including," "comprises," "comprising," or any other variation thereof, are intended to cover a non-exclusive inclusion, such that a process, method, article, or apparatus that comprises a list of elements does not include only those elements but may include other elements not expressly listed or inherent to such process, method, article, or apparatus. An element preceded by "comprises a . . . " does not, without more constraints, preclude the existence of additional identical elements in the process, method, article, or apparatus that comprises the element.

Referring to FIGS. 1-18, reference numeral 10 generally designates a monitoring system for tracking pressure injury risk that includes at least one support apparatus 12 having a controller 14. The controller 14 is configured to communicate use data or information relating to the support apparatus 12. Communication circuitry 16 is operably coupled to the support apparatus 12 and is configured to communicate via a communication network 18. A control unit 20 is configured to communicate with at least one of a remote server 22 and the support apparatus 12 via the communication network 18. The control unit 20 is configured to determine at least one of a risk status and a risk score for developing a pressure injury in response to communication with at least one of the remote server 22 and the support apparatus 12. The control unit 20 is configured to generate a notification 24 configured to be communicated to a user interface assembly 26.

Referring to FIGS. 1-3, the monitoring system 10 is configured to retrieve information from a variety of information sources 30 to determine the risk status, the risk score, or both of a patient during the stay at a medical facility 40. This information may also be referred to as the use information and may include data about the use of various devices, time of use, type of use, sensed information about the patient, etc. The patient at the medical facility 40 may be transported between several areas or units while at the medical facility 40. For example, the patient may be transferred between different departments or units on different floors 40A-40D within the medical facility 40 depending on a treatment or procedure to be received. The patient may be transported between a room environment 42, a surgical suite 44, imaging environments, etc. When the patient is transported between the different areas and rooms of the medical facility 40, different caregivers or caregiving teams may care for the patient. The monitoring system 10 is configured to retrieve information from the various caregiving teams and units to monitor the risk of pressure injury development during the stay at the medical facility 40.

The various information sources 30 may provide information that affects whether the patient is more likely to develop a pressure injury and a treatment process provided by the caregiver. The information sources 30 may include, but are not limited to, treatment devices 56, the remote server 22, electronic medical records (EMR) 46, facility risk protocols 48, a remote device 50, a facility connected device 52, a local server 54, the treatment devices 56, a caregiver interaction system 58, other systems of the medical facility 40, or a combination thereof.

Referring still to FIGS. 1-3, the patient may arrive at the medical facility 40 for one or more surgical procedures and be included in a perioperative environment, which includes a preoperative phase, an intraoperative phase, and a postoperative phase based on the timing relative to a surgical procedure. In the preoperative phase, a caregiver generally conducts a pressure injury assessment to determine a risk of the patient developing a pressure injury.

A pressure injury is localized damage to the skin and underlying soft tissue. Generally, the pressure injury is developed over a bony prominence and may be related to the use of the medical or treatment device 56. Pressure injuries develop as a result of intense pressure, prolonged pressure, pressure in combination with shear, or a combination thereof. There are stages of severity of pressure injuries. These stages include stage one of non-blanchable erythema of intact skin, stage two of partial-thickness skin loss with exposed dermis, stage three of full-thickness skin loss, stage four of full-thickness skin and tissue loss, and unstageable pressure injury of obscured full-thickness skin and tissue loss. For example, the pressure injury may present as intact skin, an open ulcer, purple discoloration, or blistering of the skin within 48 to 72 hours after a surgical procedure. A variety of factors contribute to the tolerance of the soft tissue for pressure and shear, including, microclimate, nutrition, perfusion, comorbidities, and the condition of the soft tissue.

The intraoperative pressure injury may be a deep tissue injury that is discovered post-surgery within a subsequent care environment (e.g., a medical/surgical unit, an intensive care unit, other postoperative care, a stepdown unit, at home, etc.). Additionally or alternatively, the medical or treatment device 56 used by the caregiver may cause a pressure injury, which is generally referred to as a medical device related pressure injury (e.g., a type of pressure injury). A medical device related pressure injury generally results from the use of devices designed and applied for diagnostic or therapeutic purposes. The pressure injury may conform to the pattern of shape of the device. A subset of the medical device related pressure injury is a mucosal membrane pressure injury, which is found on mucous membranes with a history of medical device use at the location of the pressure injury (e.g., a nose, a mouth, etc.) Intraoperative pressure injuries and medical device related pressure injuries are generally obtained while the patient is at the medical facility 40 and may be discovered while in another unit of the medical facility 40 or in the subsequent care environment.

Upon the patient arriving at the medical facility 40, the caregiver may conduct an initial pressure injury assessment that provides an initial risk status or an initial risk score associated with the patient that corresponds with the likelihood of the patient developing a pressure injury. To conduct the initial assessment, the caregiver may utilize one or more risk assessment tools, such as the Braden scale, the Norton scale, the Waterlow scale, the Scott Triggers, or a combination thereof. The Braden scale generally rates sensory perception, moisture, activity, mobility, nutrition, and friction and shear of the patient on a scale between two numerical values (e.g., between one and four or between one and three for friction and shear). The score for each category is added to create a total score where a higher score corresponds to a lower risk of developing a pressure injury. The Norton scale assesses five subcategories, including physical condition, mental condition, activity, mobility, and incontinence on a scale between one and four. A higher score corresponds to a lower risk of the patient developing a pressure injury.

The Waterlow scale uses varying numerical values to assess body mass index (BMI), skin type/visual risk areas, sex, incontinence, and mobility. The Waterlow scale also includes special risk categories, such as malnutrition assessments, neurological deficits, medications, and major surgeries or traumas. The score for each category is added together to produce the risk score, where a higher value corresponds with a higher risk of the patient developing a pressure injury.

The Scott Triggers are a set of evidence-based factors identified as predictors of high risk for pressure injury development. The assessment reviews four "triggers" and whether each trigger meets a specified qualification. The triggers and associated qualifications include whether the patient is over age 62, has serum albumin levels of less than 3.5 g/L or a BMI less than 19 or greater than 40, has a physical status classification (ASA) score greater than 3, and has an estimated surgery time from entering to exiting the operating room of over 180 minutes. Two or more "yes" answers to the triggers and the surgical patient is considered at high risk of developing a pressure injury. Generally, whether using the Braden scale, the Norton scale, the Waterlow scale, or the Scott Triggers, significant factors in determining the risk score or the likelihood that a patient develops the pressure injury include the length of a surgical procedure and the related time on the support apparatus 12 during the surgical procedure. These two factors generally result in unrelieved pressure when the patient is under the physiological effects of anesthetic gas and medicines, which contributes to the development of the pressure injury.

The risk of the patient developing a pressure injury is cumulative during the time the patient is at the medical facility 40. Accordingly, time spent on the support apparatus 12 or using other treatment devices 56 throughout the treatment process all contribute to the risk of developing a pressure injury. For example, if the patient is at the medical facility 40 for a surgical procedure, time in the preoperative phase, the intraoperative phase, and the postoperative phase before being discharged to the subsequent care all contribute to the risk of developing a pressure injury.

Referring still to FIGS. 1-3, upon intake of the patient, a new patient profile 60 is created, which may be entered via a computerized software program or an application on the remote device 50 utilized by caregivers. The patient profile 60 may be created through the remote device 50 (e.g., a phone, a tablet, a laptop, a wearable device, etc.) or the facility connected device 52 (e.g., a desktop or other computer within the medical facility 40, such as at a central nurse call station). The remote device 50 and the facility connected device 52 are each in communication with the local server 54 within the medical facility 40. Accordingly, using either the remote device 50 or the facility connected device 52, the patient profile 60 may be created and communicated to and at least partially stored in the local server 54. The patient profile 60 may also be at least partially stored within the remote server 22 without departing from the teachings herein. Each caregiver associated with the patient is added or granted access to the patient profile 60 to review, add, or remove information in the patient profile 60. Additional users may be manually added to the patient profile 60, and the users may be searched within the software or application by name, role, unit, or team assignments.

Both the remote device 50 and the facility connected device 52 may include an application or software program associated with and configured to interact with software on the local server 54. The software of the local server 54 may include the caregiver interaction system 58 to provide a communication platform for connecting each caregiver associated with the patient. For example, the caregiver associated with the patient in the preoperative phase, a surgical team associated with the patient in the intraoperative phase, and a caregiver associated with the patient in the postoperative phase may all communicate about the status of the patient and the related treatment through the software on the various devices and servers. Further, the software allows the caregivers to maintain and update the history of the patient across different shifts of caregivers and treatments within the medical facility 40. The software for the caregiver interaction system 58 may be advantageous for communicating information that may not usually be stored within medical or health records (e.g., the EMR 46) of the patient.

The interconnectedness of the devices and servers may be advantageous for reporting a variety of information regarding the patient during the perioperative stay or treatment process of the patient at the medical facility 40. Accordingly, the remote device 50, the facility connected device 52 (such as a room computer or a computer at a nurse station), and the local server 54 each include the software for accessing the patient profile 60, creating an interconnected network of caregivers associated with the patient. The interconnected network extends through the medical facility 40 and may extend beyond the medical facility 40 to other caregivers at other facilities associated with the patient.

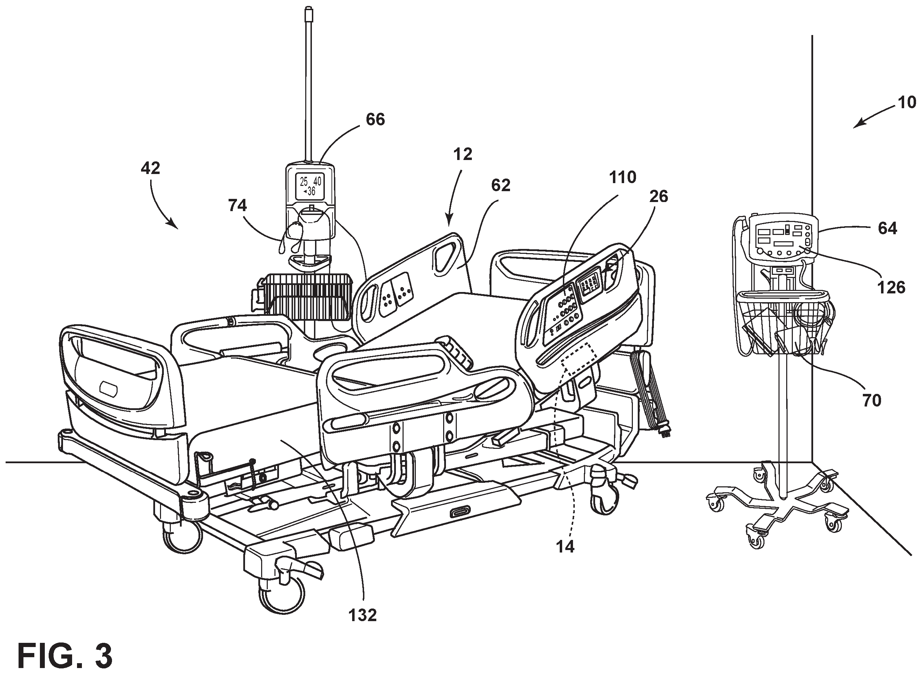

Referring still to FIGS. 1-3, as well as FIG. 4, a variety of treatment devices 56 may be utilized in the perioperative environment and/or for providing treatment to the patient at the medical facility 40. For example, as illustrated in FIG. 3, the room environment 42 may include multiple treatment devices 56, such as the support apparatus 12 configured as a medical bed 62, a vital signs monitor 64, and an oxygen therapy device 66. Based on the treatment or surgical procedure, the patient may be positioned on the medical bed 62 for a period of time during the stay at the medical facility 40, which may contribute to the development of a pressure injury.

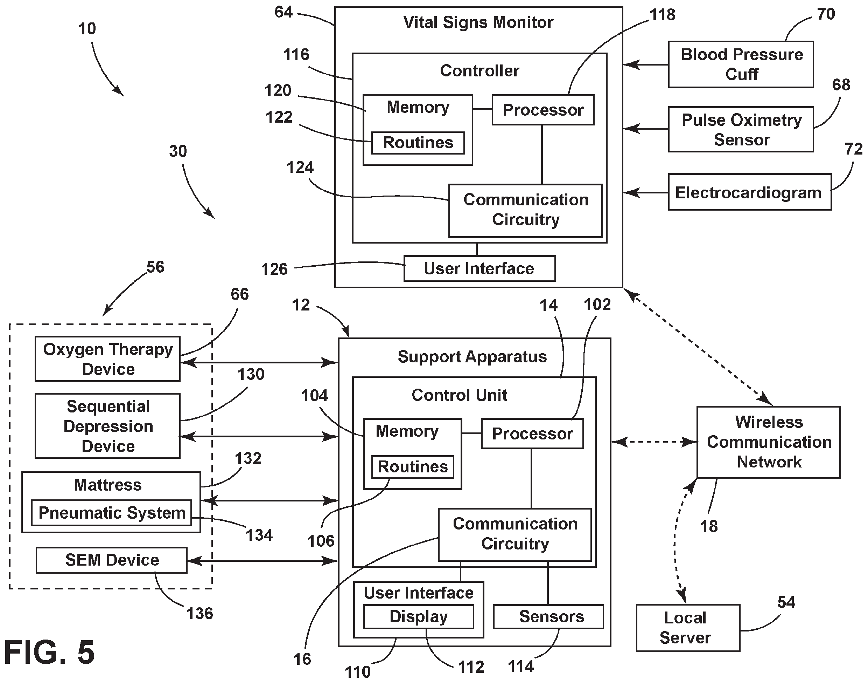

Additionally or alternatively, various vital signs of the patient may be monitored through the vital signs monitor 64. For example, a pulse oximetry sensor 68 (FIG. 5) may be utilized to determine a blood oxygen level or SpO₂ of the patient. Generally, the pulse oximetry sensor 68 is configured as a finger clasp worn by the patient. Additional devices worn or otherwise utilized to obtain vital signs data include, for example, a blood pressure cuff 70 and an electrocardiogram 72. Additional monitors associated with the vital signs monitor 64 may include, for example, electroencephalographs, heart rate monitors, respiration rate monitors, temperature monitors, blood pressure monitors, and the like. Further, additional treatment devices 56 may include an incontinence sensor, a mobility sensor, a nasal cannula 74 (e.g., oxygen tubing) associated with the oxygen therapy device 66, oxygen tubing for ears, a Foley catheter, a cast, a splint, a cervical collar, a continuous positive airway pressure (CPAP) mask, a bilevel positive airway pressure (BiPAP) mask, an endotracheal tube, a feeding tube, a halo brace, and a tracheostomy neck plate. Each of these treatment devices 56 may be utilized for a period of time, which may contribute to a pressure injury depending on the patient, the treatment, and the risk score of the patient.

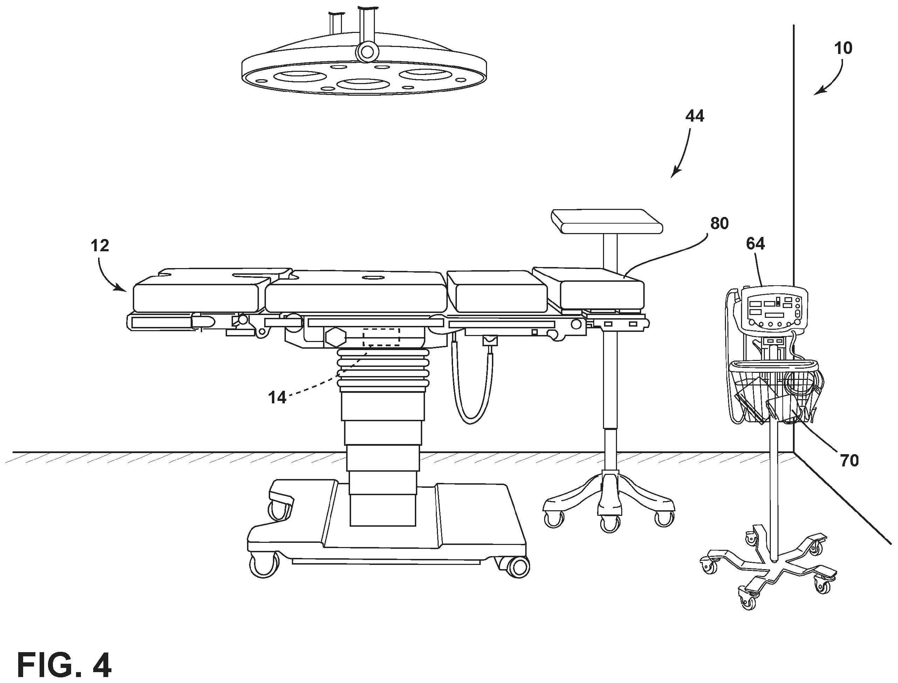

As best illustrated in FIG. 4, if the patient undergoes one or more surgical procedures additional or alternative treatment devices 56 may be utilized in the surgical suite 44. For example, the vital signs monitor 64 may be used in the surgical suite 44 to monitor the vital signs of the patient during the surgical procedures. Additionally, the patient is generally positioned on the support apparatus 12, which is configured as a surgical table 80 for use during the one or more surgical procedures. The patient may be disposed in a certain position (e.g., a prone position, etc.) on the surgical table 80 to create greater access to a surgical site for the surgical procedure. Certain positions on the surgical table 80 and extended time in those positions are more likely to result in pressure injuries to the patient. The use of one or more of the treatment devices 56 may be monitored and tracked by the caregivers to update the risk score of the patient during the treatment process of the patient at the medical facility 40.

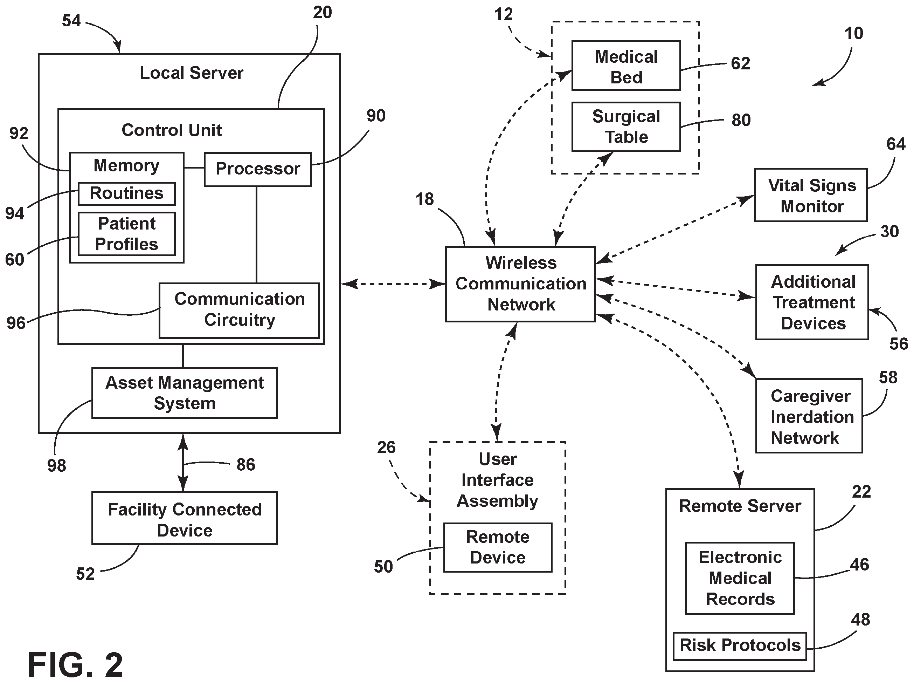

Referring again to FIG. 2, the communication between various information sources 30, including the local server 54, the support apparatus 12, the vital signs monitor 64, the additional treatment devices 56, the remote device 50, and the remote server 22 via the communication network 18 is illustrated. The communication network 18 may be part of a network of the medical facility 40 (FIG. 1). The network may include a combination of wired connections (e.g., Ethernet 86) as well as wireless networks, which may include the wireless communication network 18. The communication network 18 may include a variety of electronic devices, which may be configured to communicate over various wired or wireless communication protocols.

In the illustrated configuration of FIG. 2, the remote device 50 may be in wireless communication with each of the treatment devices 56 and provide for remote access and control of the treatment devices 56. The communication network 18 may include a wireless router through which the remotely accessed treatment devices 56 and the remote device 50 may be in communication with one another, as well as the local server 54 or the remote server 22 via the communication network18.

The communication network 18 may be implemented via one or more direct or indirect, non-hierarchical communication protocols, including but not limited to Bluetooth^{®}, Bluetooth^{®} low-energy (BLE), Thread, Ultra-Wideband, Z-wave, ZigBee, etc. Additionally, the communication network 18 may correspond to a centralized or hierarchal communication interface where one or more of the treatment devices 56 or the remote device 50 communicate via the wireless router (e.g., a communication routing controller).

Accordingly, the communication network 18 may be implemented via a variety of communication protocols in various combinations, including, but not limited to, global system for mobile communication, general packet radio services, code division multiple access, enhanced data GSM environment, fourth-generation (4G) wireless, fifth-generation (5G) wireless, Wi-Fi, world interoperability for microwave access, WiMAX, local area network, Ethernet 86, etc. By flexibly implementing the communication network 18, the various treatment devices 56, the support apparatus 12, the vital signs monitor 64, and the remote device 50 may be in communication with one another and the remote server 22 directly via the wireless communication network 18 or via a cellular data connection.

Referring still to FIG. 2, the control unit 20 of the monitoring system 10 is illustrated in the local server 54, though it is contemplated that the monitoring system 10 may be partially in the local server 54, the remote server 22, or a combination thereof. The control unit 20 includes a processor 90, a memory 92, and other control circuitry. Instructions or routines 94, including the software associated with the patient profile 60, are stored in the memory 92 and executable by the processor 90. The established patient profiles 60 may also be stored within the memory 92 of the control unit 20. The memory 92 may be implemented in a variety of volatile and nonvolatile memory formats. The control unit 20 may include various types of control circuitry, digital or analog, and may include the processor 90, a microcontroller, an application specific integrated circuit (ASIC), or other circuitry configured to perform various inputs or outputs, control, analysis, and other functions described herein. The control circuitry may also include communication circuitry 96 to permit communication via the communication network 18 or various protocols for wireless communication in combination with a wired network. The routines 94 may include operating instructions to enable the various methods described herein.

Additionally or alternatively, the control unit 20 may be in communication with an asset management system 98, which may be included in the local server 54. The asset management system 98 may track materials at the medical facility 40 and monitor when stocks or inventory are low and materials should be ordered. When the caregivers utilize treatment materials and when the medical facility 40 receives treatment materials, the information is input to the asset management system 98, allowing the asset management system 98 to track the treatment materials utilized in various prevention treatments, including those associated with pressure injuries. The monitoring system 10 may also communicate various treatment materials utilized for treating or preventing pressure injuries for monitoring by the asset management system 98.

Referring to FIG. 5, each support apparatus 12 may include the controller 14 that has a processor 102, a memory 104, and other control circuitry. Instructions or routines 106 are stored within the memory 104 and executable by the processor 102. The support apparatus 12 may also include the communication circuitry 16 for communicating information and data relating to use of the support apparatus 12 via the communication network 18. The memory 104 may be implemented in a variety of volatile and nonvolatile memory formats. The controller 14 may include various types of control circuitry, digital or analog, and may include the processor 102, a microcontroller, an ASIC, or other circuitry configured to perform various inputs or outputs and other functions described herein.

The support apparatus 12 may also include a user interface 110 that is included in the user interface assembly 26. The user interface 110 may display a variety of information relating to the support apparatus 12, the patient, the risk information relating to development of the pressure injury, etc. The user interface 110 may include a display 112 to convey information, as well as receive various inputs from the caregiver or the patient. The user interface 110 may be included in any practicable location on the support apparatus 12, such as on a siderail or a pendant.

In various aspects, the support apparatus 12 includes sensors 114 for monitoring information about the support apparatus 12 and the patient thereon. For example, the sensors 114 may monitor the position of the support apparatus 12. In additional non-limiting examples, the sensors 114 may sense physiological data, such as heart rate and respiration rate, of the patient through non-contact monitoring. In further non-limiting examples, the sensors 114 may monitor the position and movement of the patient on the support apparatus 12. The information about the patient and the support apparatus 12 may be utilized by the monitoring system 10 to determine the likelihood of developing a pressure injury or the likely cause of a pressure injury.

Similarly, the vital signs monitor 64 may include a controller 116 having a processor 118, a memory 120, and other control circuitry. Instructions or routines 122 are stored with the memory 120 and executable by the processor 118. The memory 120 may be implemented in a variety of volatile and nonvolatile memory formats. The controller 116 may include various types of control circuitry, digital or analog, and may include the processor 118, a microcontroller, an ASIC, or other circuitry configured to perform various inputs or outputs and other functions described herein.

The vital signs monitor 64 is in communication with the pulse oximetry sensor 68, the blood pressure cuff 70, the electrocardiogram 72, and any other sensors or devices for obtaining one or more vital signs of the patient. The vital signs monitor 64 includes communication circuitry 124 for communicating use data or information as well as vital signs data via the communication network 18. The vital signs monitor 64 also includes a user interface 126 that is included in the user interface assembly 126 for displaying information to the caregiver or patient, receiving an input, or a combination thereof.

Other treatment devices 56 may include a user interface, a controller, and other similar features associated with the monitoring system 10 as described relative to the support apparatus 12 and the vital signs monitor 64. For example, additional treatment devices 56 including the oxygen therapy device 66, a sequential compression device 130, a mattress 132 including a pneumatic system 134, and a sub-epidural moisture (SEM) device or scanner 136 in the form of a scanner. Each treatment device 56 may communicate directly with the support apparatus 12 or may have communication circuitry to communicate via the communication network 18.

The oxygen therapy device 66 provides oxygen to the patient through the nasal cannula 74. The sequential compression device 130 may generally be configured as a sleeve or mattress pad that includes actuators that sequentially activate to drive fluid or blood in a selected direction (e.g., toward a central cavity of the body). The sequential compression device 130 may be used for the treatment of lymphedema, deep vein thrombosis, or other conditions. The mattress 132 may include the pneumatic system 134 that has a plurality of bladders associated with a pump, which controls fluid directed into and out of the bladders. The pneumatic system 134 may adjust a pressure of the mattress 132 as well as provide therapies such as alternating pressure therapy or vibrating or percussion therapies. The SEM scanner 136 may provide information relating to the presence of an edema or change in electrical capacitance of tissue, such as sub-epidermal moisture around bony prominences. The illustrated or described treatment devices 56 are exemplary and are not meant to be limiting.

As illustrated in FIG. 5, the additional treatment devices 56 are associated with the support apparatus 12. In such configurations, the treatment devices 56 are configured to communicate with the support apparatus 12. Generally, the additional treatment devices 56 may be directly coupled with the support apparatus 12. The support apparatus 12 may then communicate the data from the additional treatment devices 56 and the support apparatus 12 to the local server 54.

It is also contemplated that the additional treatment devices 56 may be separate from the support apparatus 12 and configured to communicate via the communication network 18 in a similar manner as the support apparatus 12 and the vital signs monitor 64, as set forth herein. It is also contemplated that certain treatment devices 56 may not be configured to communicate use information. Use information from these treatment devices 56 may be manually inputted into the patient profile 60 by the caregiver through the application.

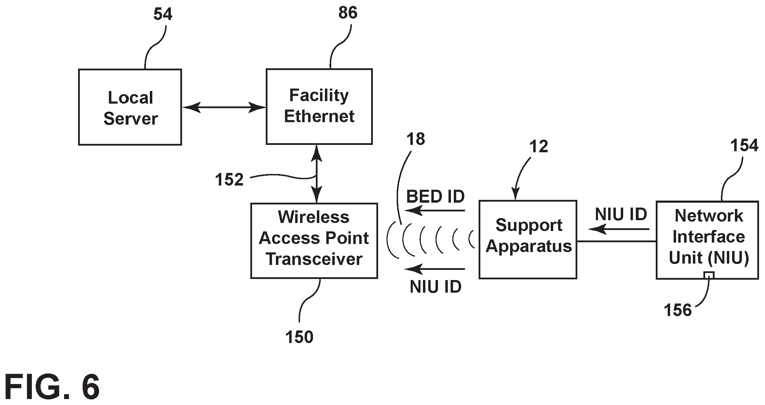

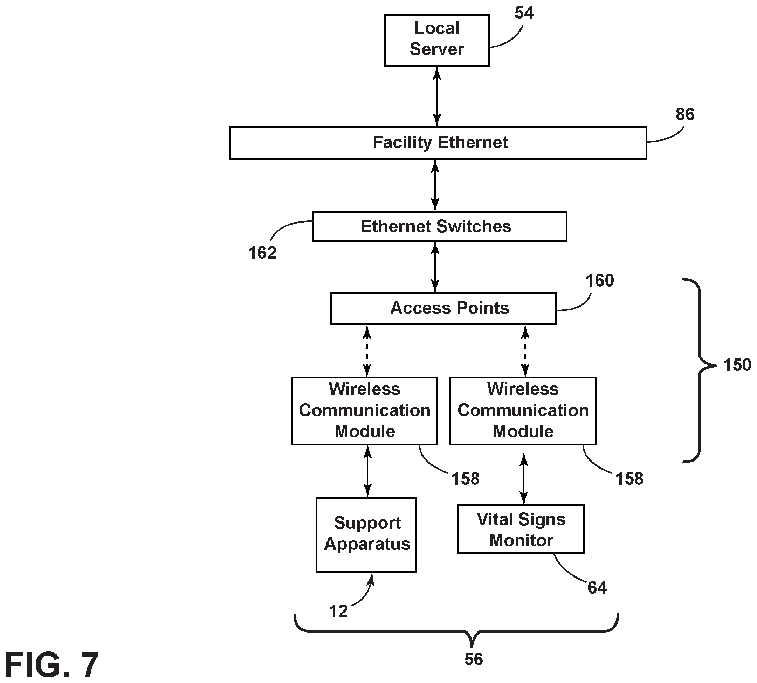

Referring still to FIG. 6, as well as FIG. 7, in certain aspects, some or all of the treatment devices 56 are configured to communicate with a wireless access transceiver 150, which is coupled to Ethernet 86 of the medical facility 40. The communication network 18 provides for bidirectional communication between the treatment device 56 and the wireless access transceiver 150. In the example illustration, the treatment device 56 is illustrated as the support apparatus 12 but may be any practicable treatment device 56. The wireless access transceiver 150 communicates bidirectionally with Ethernet 86 via a data link 152.

The support apparatus 12 may be associated with a network interface unit 154. Multiple network interface units 154 may be provided in various locations within the medical facility 40. Each support apparatus 12 and each network interface unit 154 is assigned a unique identification (ID) code such as a serial number. Various components of the monitoring system 10 (e.g., the local server 54, the remote device 50, the facility connected device 52, or a combination thereof) may include software (e.g., routines) that operates to associate the ID of the support apparatus 12 with the network interface unit ID data to locate each support apparatus 12 within the medical facility 40. Each network interface unit 154 includes a port 156 for selectively coupling with Ethernet 86. When the network interface unit 154 is coupled with Ethernet 86, the network interface unit 154 communicates the ID data to the support apparatus 12, which then wirelessly communicates the ID data for the support apparatus 12 and the network interface unit 154 to the wireless access transceiver 150.

Referring still to FIG. 7, some or all of the treatment devices 56 within the medical facility 40 may be capable of communicating wirelessly via a wireless communication module 158. In the illustrated configuration of FIG. 7, which is exemplary and not meant to be limiting, the treatment devices 56 are illustrated as the support apparatus 12 and the vital signs monitor 64. It is contemplated that each treatment device 56 disclosed herein may be configured to operate in a similar manner to communicate wirelessly, as set forth in further detail herein.

The wireless communication modules 158 generally communicate via an SPI link with circuitry of the associated support apparatus 12 (e.g., the communication circuitry 16) and circuitry of the vital signs monitor 64 (e.g., the communication circuitry 124) and via a wireless 802.11b link with wireless access points 160. The wireless access points 160 are generally coupled to Ethernet switches 162 via 802.3 links. It is contemplated that the wireless communication modules 158 may communicate with the wireless access points 160 via any of the wireless protocols disclosed herein. Additionally or alternatively, the Ethernet switches 162 may generally communicate with Ethernet 86 via an 802.3 link. Ethernet 86 is also in communication with the local server 54, allowing information and data to be communicated between the local server 54 and the various treatment devices 56.

Referring to FIGS. 1-7, when the treatment devices 56, including the support apparatus 12, the vital signs monitor 64, and the additional treatment devices 56, are coupled to the local server 54, each may wirelessly transmit use data, directly or indirectly. The use data or information may include the duration of use, type of use, therapies applied, patient data (e.g., position, pressures, physiological information, movement, etc.), or any other data useful to the caregiver. The data from the treatment devices 56 may be routed to different remote devices 50 and facility connected devices 52 or to different software programs within the respective device or server. The data may also be communicated to the remote server 22 to be included or stored in the EMR 46 associated with the patient. The wireless communication between the various treatment devices 56 may be at different frequencies or different times. Accordingly, communication circuitry of the various treatment devices 56 may operate independently of one other.

It will be understood that the use data, the patient data, or other data received from the local server 54 may be displayed on the user interface assembly 26. The user interface assembly 26 includes, for example, the remote device 50, the facility connected device 52, the user interface 110 of the support apparatus 12, and the user interface 126 of the vital signs monitor 64. This provides a variety of places the caregiver can observe information about the patient, including the risk information. Accordingly, the user interface assembly 26 allows for continued updates to the caregiver regarding the risk of developing a pressure injury.

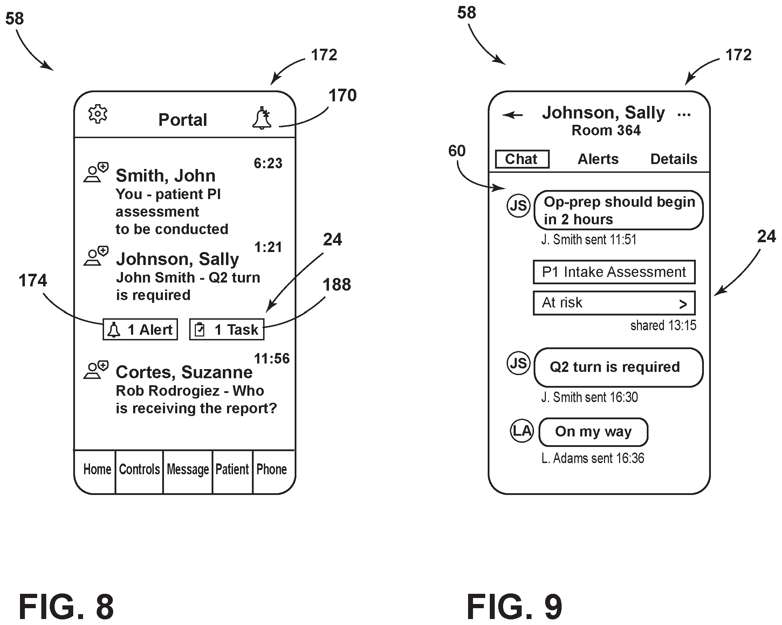

Referring to FIGS. 8 and 9, as well as FIGS. 1-7, the application or software operating on the remote device 50 may provide for communication between multiple remote devices 50 associated with different caregivers, the facility connected device 52 or devices, the local server 54, and the remote server 22 via the caregiver interaction system 58. A graphical representation of an application interface 172 displayed on the user interface assembly 26 is illustrated in FIG. 8. Each user interface of the user interface assembly 26 may have a similar configuration as the application interface 172 based on the application or software operating on the respective device. Alternatively, the configuration of the application interface 172 may differ based on the specific configuration of the user interface, while displaying the information and data sought by the caregiver. It is contemplated that each patient profile 60 may include the risk information, details of the patient, chat messages between caregivers, etc.

The application interface 172 is configured to display a communication portal 170 for each caregiver including multiple patient profiles 60, with each patient profile 60 corresponding to one patient associated with the caregiver. Each caregiver associated with the patient is granted access to the respective patient profile 60, which can be accessed through the communication portal 170. Additionally or alternatively, the communication portal 170 provides a way for each caregiver associated with the patient to communicate with one another, as well as a way to receive updates about the patients.

Referring still to FIG. 9, an exemplary patient profile 60 is illustrated on the application interface 172. The patient profile 60 allows for messaging and sharing information (e.g., a chat feature) relating to the patient between various caregivers throughout the treatment process of the patient at the medical facility 40. Accordingly, the caregivers can directly message one another through the patient profile 60 throughout the preoperative phase, the intraoperative phase, the postoperative phase, or any other treatment process at the medical facility 40. The caregivers can discuss the treatment of the patient, request tasks to be done, and share information about the status of the patient or the treatment.

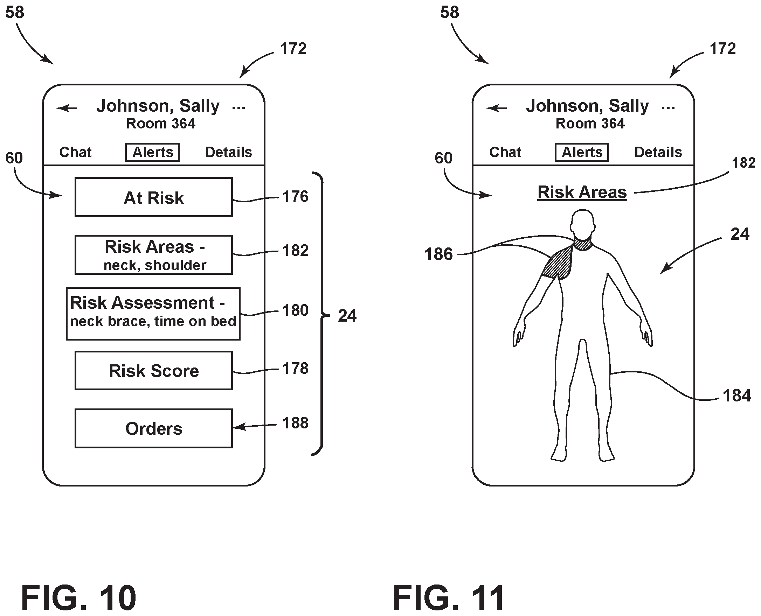

Referring again to FIG. 8, as well as FIG. 10, the communication portal 170 may indicate when there is a risk alert 174 regarding a certain patient. The caregiver may select the alert icon to view the alert information, as illustrated in FIG. 10. The application interface 172 may then provide the information relating to the risk alert 174. For example, if a patient is deemed at risk of developing a pressure injury through a surgical procedure, use of the medical or treatment device 56, or another method, the application interface 172 provides a way to communicate the risk information to each caregiver associated with the patient profile 60.

As best illustrated in FIG. 10, the application interface 172 communicates a variety of risk information and data of the patient to the caregiver. In the exemplary configuration of FIG. 10, the application interface 172 displays the notification 24, which includes a risk status indicator 176 that communicates to the caregiver whether the patient is considered at risk of developing a pressure injury (e.g., the risk status), a risk score indicator 178 determined through the methods described in further detail herein and/or the initial risk assessment as described herein, etc.

Additionally, the application interface 172 may display a risk assessment 180 of the patient, including the factors that contribute to the risk score, the risk status, and the likelihood of the patient developing a pressure injury. Further, the application interface 172 may communicate risk areas 182 to the caregivers, thereby allowing the caregiver to monitor areas of the body of the patient that are more likely to develop a pressure injury based on the risk assessment 180. Each of these may be selectable features that cause the control unit 20 to generate a subsequent or updated notification 24 upon a selection. Generally, the application interface 172 communicates information to each caregiver associated with the patient to allow the caregivers to provide prevention treatment or measures and monitor the patient for pressure injuries. The risk information and data may be updated throughout the perioperative or treatment process of the patient.

Referring still to FIG. 10, as well as FIG. 11, the application interface 172 may provide an icon or graphical representation 184 of the risk areas 182 of the patient. For example, the notification 24 includes a graphic or icon of a person. The notification 24 also includes an identifier 186, such as an arrow, a shape, a highlighted area, etc., on the icon that corresponds with the risk areas 182. The identified areas 182 may be automatically generated by the control unit 20. Additionally or alternatively, the caregiver may highlight or otherwise indicate various areas 182 that are determined to be at risk of developing a pressure injury. The graphical representation 184, as well as other risk information, may also be included in the EMR 46 (FIG. 2) of the patient. As the control unit 20 receives additional information, the risk areas 182, as well as the graphical representation 184 in the notification 24, may be updated and communicated to the caregiver.

Referring still to FIGS. 10 and 11, as well as FIG. 12, the notification 24 displayed on the application interface 172 may present tasks or treatment orders 188 that relate to prevention treatment for the patient in response to the risk information (e.g., risk status, risk score, risk assessment 180, risk areas 182, etc.). The treatment orders 188 may be generated and displayed in response to the selection of an icon for the treatment orders 188 on the application interface 172 (e.g., as illustrated in FIG. 8). The treatment orders 188 may be tailored to the specific risk areas 182 or risk factors of the patient and may be generated by the control unit 20. The risk factors are generally factors utilized by the monitoring system 10 to determine how and why the patient likely developed the pressure injury.

In the treatment orders 188, the caregiver is provided with a specific listing of tasks for treating the patient to prevent or minimize a potential pressure injury from developing. The treatment orders 188 may include, but are not limited to, Q2 turns (e.g., turning the patient at two hour intervals to keep the patient from lying on a risk area 182), use of an offloading device (e.g., a heel boot, etc.), recommendation for an offloading position (e.g., place the patient on the left side as patient spent a certain amount of time on the right side), order or upgrade a therapeutic surface to a specialty mattress 132, consult a wound, ostomy, and incontinence nurse (WOCN), apply barrier creams, utilize anti-shear and friction pads (e.g., Mepilex^{®} pads), institute incontinence management, promote microvascularization, etc.

The application interface 172 may be advantageous for quickly sharing information between caregivers. For example, one caregiver may view the treatment orders 188 and may "share" the treatment orders 188 through the chat feature of the patient profile 60, as illustrated in FIG. 9. Additionally, the caregiver who conducts a task or treatment order 188 can mark the treatment order 188 as completed to communicate to the other caregivers what actions have been completed and what tasks are still incomplete. In this way, the control unit 20 may update the notification 24 with completion identifiers 190 (e.g., checks, strikethrough, etc.) that correspond with the completed tasks.

The treatment orders 188 and the risk alert 174 may be provided through the application or software acting on any of the remote devices 50, the facility connected device 52, the user interface assembly 26, or a combination thereof. Additionally, this information may also be communicated to the EMR 46 (FIG. 2) associated with the patient and stored therein. Accordingly, when the caregiver accesses the application interface 172 or EMR 46, the caregiver is alerted of the risk status and associated tasks or treatment orders 188 for the patient. The treatment orders 188 may also include more general tasks for preventing pressure injuries that may not be specific to the patient. The treatment orders 188 may be directed by the facility risk protocol 48 (FIG. 2) stored in the remote server 22.

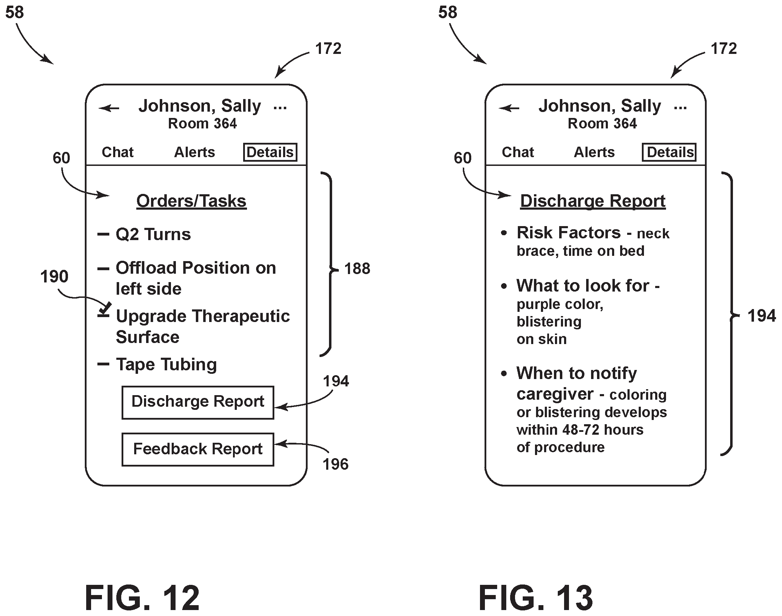

Referring to FIG. 13, the notification 24 displayed on the application interface 172 may provide for viewing or updating a discharge report 194. The caregiver may manually generate or update the discharge report 194. Additionally or alternatively, the control unit 20 may automatically generate or update the discharge report 194, or a portion of the discharge report 194, to include information and education related to the potential pressure injury and the risk of the patient of developing the pressure injury. The discharge report 194 may be automatically updated based on the risk status, the risk score, observations of the caregiver, etc. input into the monitoring system 10. The application interface 172 may include a selectable feature that, when selected, causes the control unit 20 to generate the updated notification 24 including the discharge report 194.

The discharge report 194 may include risk factors, information or data on how to identify a pressure injury, information relating to when to notify the caregiver about a potential pressure injury, information on contacting the caregiver for pressure injury related treatment, or any educational or other information useful for the patient or subsequent caregiver. Accordingly, the monitoring system 10 assists in providing education to the subsequent caregiver or the patient after discharge. The information included in the discharge report 194 may be updated or altered based on where the patient is being discharged to (e.g., within the medical facility 40 compared to home) and the patient.

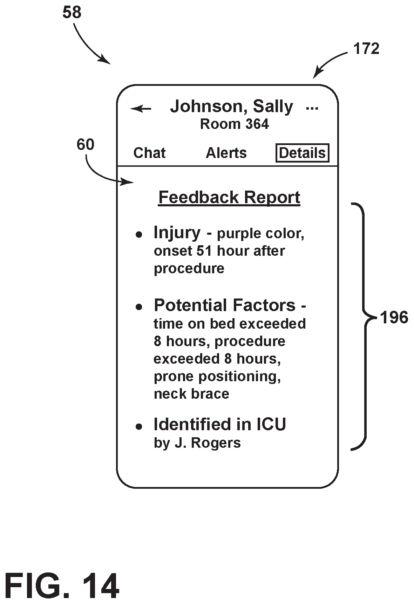

Referring to FIG. 14, the application interface 172 may also provide a feedback report 196 that is communicated to each caregiver associated with the patient when the patient has developed a pressure injury. Generally, the control unit 20 generates the feedback report 196 as discussed in further detail herein. The feedback report 196 provides, for example, information relating to the risk score, a listing of risk factors, details of the pressure injury, potential causes or contributing factors of the pressure injury, identification of the pressure injury, or any other information useful to the caregiver. Additional information that may be useful to the caregivers may also be included in the feedback report 196, such as, for example, details on the pressure injury, details of the location of the pressure injury, etc.

As previously stated, the risk factors are generally factors utilized by the monitoring system 10 to determine how and why the patient likely developed the pressure injury. In other words, the risk factors are contributing factors related to the development of the pressure injury. These contributing risk factors include, for example, characteristics of the pressure injury, length of time on the support apparatus 12, length of treatment, a procedure or diagnosis, treatment devices 56 utilized, areas the treatment devices 56 were utilized, or a combination thereof. The monitoring system 10 utilizes the risk factors to determine a likely cause of the pressure injury and conveys the likely cause, along with the listing of risk factors, to the caregivers. The likely cause may include specific equipment and the monitoring system 10 may associate the equipment with a certain perioperative phase (e.g., the surgical table 80 in the surgical suite 44). The monitoring system 10 may also include a time or phase of the perioperative environment, which was the likely cause of the pressure injury without specific equipment. In this way, the monitoring system 10 provides an explanation in the feedback report 196 for a determined cause of the pressure injury and for how the monitoring system 10 determined the likely cause of the pressure injury.

Accordingly, the monitoring system 10 provides feedback to each caregiver and each caregiving team regarding the status of the patient in developing a pressure injury. In certain aspects, an operating team for the patient also receives the feedback report 196, which may be advantageous for alerting the operating team if the pressure injury was likely caused in the surgical suite 44 or by a specific treatment device 56. The feedback report 196 may also be stored in the EMR 46 (FIG. 2) of the patient.

Accordingly, each caregiver may be alerted of the feedback report 196 when accessing the application interface 172 (including in the communication portal 170) and the EMR 46 of the patient. The feedback report 196 assists in monitoring the health of the patient as well as monitoring actions taken by the caregiving team that contributed to the pressure injury. In this way, the feedback reports 196 may be utilized to improve the care provided to the patient. The information in the feedback report 196 may be updated based on the type of injury, the severity of the injury, timing of the onset of the injury, type of procedure, type of treatment device 56 utilized, etc.

The feedback report 196 may also include facility risk protocols 48. Depending on the information provided to the monitoring system 10, the feedback report 196 may also include whether actions of the caregiving team followed or deviated from the facility risk protocols 48, which may have affected the development of the pressure injury. The feedback report 196 with the facility risk protocols 48 may also assist in updating the facility risk protocols 48 over time.

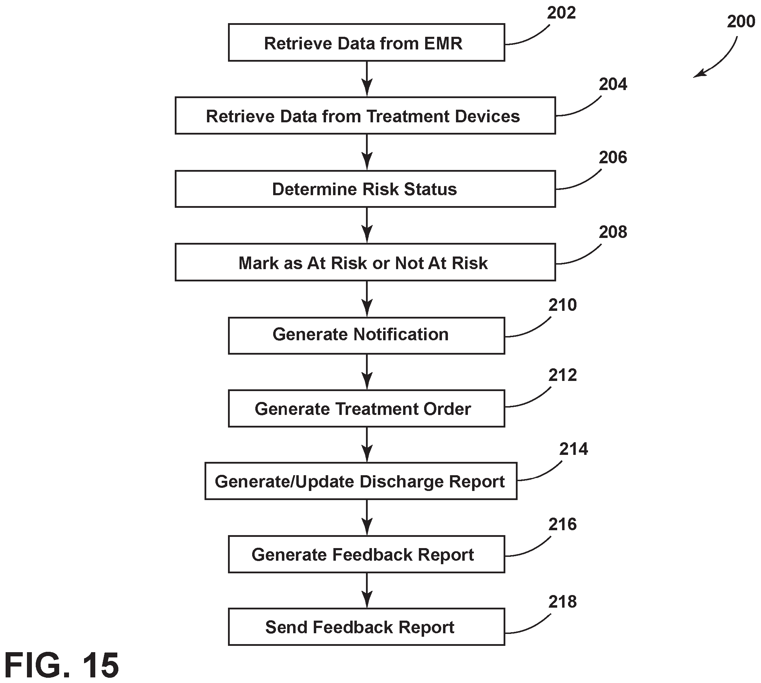

Referring to FIG. 15, as well as FIGS. 1-14, the control unit 20 may perform various methods for tracking and monitoring a risk associated with the patient developing a pressure injury, communicating information relating to the factors that contribute to the risk, communicating educational information to the caregiver and the patient, and communicating the development of a pressure injury to the caregiver. The control unit 20 may operate with a rules-based software, a predictive software, or a combination thereof. Further, the control unit 20 may also perform methods directed to pressure injuries obtained in the perioperative process, medical device related pressure injuries, or a combination thereof.

For example, as illustrated in FIG. 15, the control unit 20 of the monitoring system 10 may perform a method 200 for monitoring and tracking the risk and/or development of a pressure injury through operating a rules-based software. In step 202 of the method 200, the control unit 20 collects or retrieves data from the EMR 46 of the patient. The information may be scraped from notes within the EMR 46 via natural language processing. The control unit 20 communicates with the remote server 22 via the communication network 18.

Generally, the data or information retrieved from the EMR 46 relates to patient demographics. For example, information collected from the EMR 46 may include, but is not limited to, age, history of pressure injuries, procedure or treatment to be performed or that has already been performed, comorbidities such as diabetes, malnutrition, any vascular issue, smoking history, patient weight, patient BMI, nothing by mouth (NPO) status, medications, the risk score obtained at the initial intake, any subsequent risk scores, data from another unit or home care, data from home care equipment, lab data such as hemoglobin, hematocrit, creatine, and other lab data that captures a current hydration status, etc.

In step 204, the control unit 20 collects or retrieves use data or information from the various treatment devices 56 utilized by or for the patient at the medical facility 40 in the perioperative environment. For example, the control unit 20 may retrieve data from preoperative and postoperative devices, such as the medical bed 62 or the surgical table 80, to obtain information regarding the period of time the patient was positioned on the medical bed 62. The support apparatus 12 may include the sensors 114 that sense a variety of information and communicate the information to the control unit 20. The sensors 114 may be configured to sense movement of the patient, physiological data of the patient, position of the patient on the support apparatus 12, position of the support apparatus 12, etc. Additionally, the use information may be manually inputted into the remote device 50 or the facility connected device 52 by the caregiver and communicated to the control unit 20. It is also contemplated that the control unit 20 may obtain use information from other support apparatuses 12, including those in the emergency department, a radiology department, and other areas or units of the medical facility 40.

In addition to time positioned on the various support apparatuses 12, the use information relating to the time the patient is not positioned on the support apparatuses 12 prior to the surgical procedure may also be communicated to the control unit 20. Additional information retrieved or collected by the control unit 20 in step 204 may include the time of using the blood pressure cuff 70 associated with the vital signs monitor 64 or time of use of the sequential compression device 130. Additionally or alternatively, the control unit 20 may be in communication with the SEM scanner 136, which provides information relating to the presence of edema and chance in the electrical capacitance of tissue, such as sub-epidermal moisture around bony prominences.

Further, in step 204, the control unit 20 may also obtain data from devices 56 from the intraoperative phase within the surgical suite 44 utilized during the surgical procedure. For example, the control unit 20 may obtain use information from the surgical table 80 including time on the surgical table 80 and position of the surgical table 80. Additional information that may be obtained by the control unit 20 may include, but is not limited to, the position of the patient on the surgical table 80, cumulative time spent in each position on the surgical table 80, use of positioning devices (e.g., arm boards, prophylactic pressure injury prevention dressings, etc.), micro-movements of the patient (e.g., nursing protocols where the nurse or caregiver is moving patient extremities to offload pressure, unintended movements during surgery, or sliding), use of warming devices such as blankets or specialized mattresses 132, anesthetic medication or gases used, temperature within the surgical suite 44, other treatment devices 56 in contact with the patient during the perioperative phases, etc.

This information from the surgical suite 44 may be provided directly from the selected treatment device 56 through the communication network 18. It is also contemplated that use information regarding any of the treatment devices 56 may be manually input by the caregiver through the remote device 50 or the facility connected device 52, which may then be communicated to the control unit 20 through the communication network 18 or through any of the wired or wireless protocols disclosed herein.

In step 206, the control unit 20 determines the risk status of the patient in response to the data received during steps 202 and 204 of method 200. In the rules-based software, the control unit 20 may compare data received in step 202 and step 204 with various rules or threshold factors stored within the memory 92 of the control unit 20 and determine if the rule has been satisfied. Any one or more of the inputs may be utilized as a rule by the control unit 20. The rules-based software may be based on a single rule or threshold factor. For example, when the patient is positioned on the surgical table 80 for a period of time greater than a predefined amount of time, the control unit 20 automatically determines that the patient is at risk for developing a pressure injury. Additionally or alternatively, the rules-based software may use multiple factors or conditions to determine whether the patient is at risk (e.g., if a predefined condition is satisfied or not). For example, if the patient is over a predefined age and a surgical procedure lasts longer than a predefined amount of time, the control unit 20 automatically determines that the patient is at risk. The rules utilized by the control unit 20 may be configured within the software by a rules manager and implemented within the method 200.

In step 208, the control unit 20 marks the patient as at risk or not at risk (e.g. the risk status) within the patient profile 60. This indication appears on at least one of the application interface 172 and the EMR 46. In step 210, the control unit 20 generates the notification 24 that is sent to the patient profile 60 and displayed on the application interface 172. It is also contemplated that the notification 24 is sent to the EMR 46. The notification 24 includes a variety of information relating to the risk status of the patient, as illustrated in FIGS. 10 and 11.

Additionally or alternatively, the notification 24 may be a graphical or another alert that prompts the caregiver to view the risk information on the application interface 172. In certain aspects, as illustrated in FIG. 11, the notification 24 may include a graphical schematic or the graphical representation 184 of a human body with the risk areas 182 highlighted or otherwise identified by the identifier 186 to the caregiver. When the caregiver accesses either the application interface 172 or the EMR 46, the notification 24 is automatically presented to the caregiver. The notification 24 may operate as an alert to prompt the caregiver to provide pressure injury risk assessments or care. The notification 24 may be utilized as a best practice alert to prompt the caregiver at different times in the treatment process for the patient.

Referring still to FIG. 15, in step 212, the control unit 20 generates the treatment orders 188 that are communicated to the caregiver. The treatment orders 188 may be included in the notification 24, may be accessible through the notification 24 (e.g., a selectable feature), or may be a separate alert. The treatment orders 188 generally relate to reminders to track pressure injury risk and provide preventative treatment based on the risk status of the patient, the surgical procedure, the treatment devices 56 utilized with the patient, etc. The treatment orders 188 may be displayed on the application interface 172, as illustrated in FIG. 12. The generated tasks or treatment orders 188 are generally specific to the patient based on the inputs received and risks determined by the control unit 20.

The treatment orders 188 triggered by the risk status of the patient may include, but is not limited to, Q2 turns, use of an offloading device, recommendation for an offloading position, order or upgrade a therapeutic surface to a specialty mattress 132, consult a WOCN, apply barrier creams, utilize anti-shear and friction pads, institute incontinence management, promote microvascularization, etc. The treatment orders 188 may also include treatment materials to be utilized by the caregiver to prevent or treat the pressure injury. In step 212, the control unit 20 is configured to communicate with the asset management system 98 to monitor the inventory of treatment materials at the medical facility 40. As the caregiver utilized various treatment materials, the caregiver may input information to the monitoring system 10 about the use and quantity of the treatment materials utilized. The asset management system 98 may then update and monitor the inventory or use of the treatment materials.

In step 214, the control unit 20 generates or updates the discharge report 194 for the patient or the subsequent caregiver. Updating the discharge report 194 may be advantageous for having an automatic process to include information and data related to pressure injuries for the patient or the subsequent caregiver. The discharge report 194 may include a listing of risk factors specific to the patient, information on how to identify pressure injuries including, for example, timing relative to a surgical procedure or increased risk due to a certain treatment device 56, and/or when and how to contact the caregiver related to a pressure injury, as illustrated in FIG. 13. Accordingly, the discharge report 194 may provide educational information to the patient when the patient is discharged to the subsequent care environment and/or when the patient is discharged home.

In examples when the patient is discharged home shortly after the surgical procedure, the protocol for the caregiver may include a follow-up call, visit, or other communication with the patient. The follow-up call or appointment may include questions regarding symptoms of pressure injuries to obtain the information about whether the pressure injury has developed and, if so, details of the pressure injury to be communicated to the monitoring system 10.

In step 216, if the patient develops a pressure injury, as uncovered by the caregiver or the patient, the control unit 20 automatically generates the feedback report 196. The feedback report 196 may include details of the pressure injury, details of the surgical procedure that may have contributed to the risk status of the patient, and/or other information relating to the pressure injury that may be beneficial to the caregivers, as illustrated in FIG. 14. In step 218, the feedback report 196 is sent to the caregivers, including, for example, one or more of the surgical team, a primary care physician, a surgeon, an intensive care unit, a medical/surgical unit, and an offsite care facility. The feedback report 196 may also be communicated to the EMR 46 of the patient. Accordingly, if the patient develops pressure injury due to the surgical procedure conducted at the medical facility 40, each caregiver associated with the patient is informed of the pressure injury and potential contributing factors. The feedback report 196 may be advantageous for alerting caregivers of additional prevention treatment options, as well as training to provide better care for the patient.

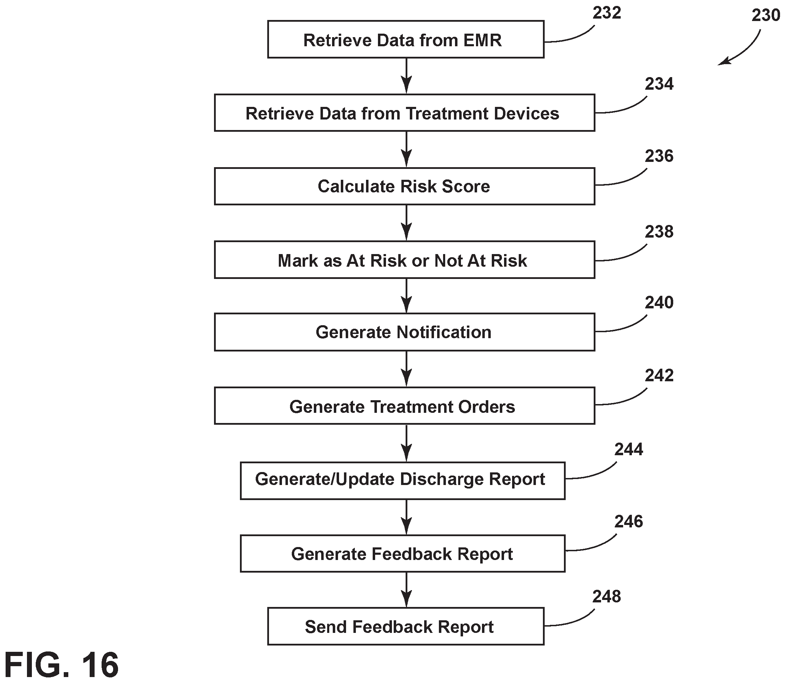

Referring to FIG. 16, as well as FIGS. 1-15, in method 230, the control unit 20 utilizes predictive software for predicting the likelihood of developing the pressure injury. The likelihood of developing the pressure injury may be quantified as a number within a range (e.g., out of 100), a percentage, a high/medium/low indication, or any other practicable scale. In step 232, the control unit 20 collects data from the EMR 46 of the patient, which is conducted in a similar manner as step 202 from method 200 as described herein. In step 234, similar to step 204 of method 200 in FIG. 15, the control unit 20 obtains use information from the various treatment devices 56 utilized by the patient. The treatment devices 56 may include one or more of the support apparatus 12 (e.g., the medical bed 62, the surgical table 80, etc.), the vital signs monitor 64, the sequential compression device 130, the SEM scanner 136, as well as other treatment devices 56 described herein or any treatment devices 56 utilized to treat or care for the patient.

In step 236, the control unit 20 calculates the risk score. When calculating the risk score, the control unit 20 compares weighted factors or inputs. The factors or inputs include the information retrieved in the previous steps of method 230. The weight assigned to each factor or input may be assigned by a rules manager of the software program. The control unit 20 may use a predictive algorithm such as, for example, logistic regression, random forest algorithm, or other machine learning models. Table 1, included below, includes exemplary factors or inputs received by manual input through the application interface 172, received directly from at least one of the treatment devices 56, the caregiver interaction system 58, other systems of the medical facility 40, or a combination thereof, which are weighted and added together to calculate the risk score.

| Table 1: Data Inputs | | | | | |
|---|---|---|---|---|---|
| EMR Data | | Pre-op / Post-op Device Data | | Intra-op Device Data | |
| • | Patient age | • | Time in bed or on a stretcher before and after surgery | • | Time on a surgical table |
| • | History of pressure injuries | | | • | Patient positions on a |
| • | Patient procedure | | | | surgical table |
| • | Comorbidities, such as diabetes, de-nutrition, vascular issues | • | Times out of bed in a preoperative environment | • | Cumulative time in each position |
| | | • | Measured micro-climate | • | Use of positioning devices |
| • | Smoking history | • | Medication administered during pre-op/post-op | • | Micro-movements of patient |
| • | Patient weight, BMI | | | | |
| • | NPO status | • | Any complications related to post-op (confusion, pain, etc.) | • | Use of warming devices |
| • | Patient medication | | | • | Anesthetic medications and gases |
| • | PI risk score from a perioperative environment | | | | |
| | | | | • | Medical device related pressure injury assessment |
| • | Lab data (Hbg/Hct, albumin, creatine, hydration-related, etc.) | | | | |
| • | Data about transfer from another unit or from home | | | | |

The control unit 20 may utilize the data received from the various inputs and calculate the risk score corresponding with the likelihood of developing the pressure injury. Additionally or alternatively, in step 236, the control unit 20 may combine the predictive algorithm with the rules-based algorithm as described herein. In such examples, the predictive algorithm weighing the various factors is utilized until a specific condition is recognized by the control unit 20. For example, the condition may be that a predetermined amount of time on the surgical table 80 has been exceeded or that the patient uses vasopressors. Once the condition is recognized by the control unit 20, the rules-based algorithm may override the predictive algorithm.

In step 238, the control unit 20 determines whether the patient is at risk or not at risk of developing a pressure injury based on the calculated risk score. The control unit 20 may store a predefined value that is compared to the calculated risk score. If the risk score is above the predefined value, the control unit 20 marks the patient as at risk and indicates the severity of the risk. Additionally, if a certain condition has been met to override the predictive algorithm, the condition may be noted with the risk score.

After calculating the risk score, the monitoring system 10 continues in a similar manner as method 200. In step 240, the control unit 20 generates the notification 24. In step 242, the control unit 20 generates treatment orders 188 for the caregiver based on the risk score. In step 244, the control unit 20 generates the discharge report 194. In step 246, the control unit 20 generates the feedback report 196, and in step 248 the feedback report 196 is sent to the caregivers associated with the patient. It is contemplated that steps 242-248 are performed in a substantially similar manner as the steps 212-218 of method 200 described herein.

Referring to FIGS. 15 and 16, the methods 200, 230 generally follow a process for monitoring and tracking general pressure injury risk in the perioperative environment. The risk is associated with an intraoperative time on the surgical table 80, as well as certain positions or positioning devices (e.g., treatment devices 56) utilized on the patient, and other time in the perioperative environment. The control unit 20 may also determine the risk status or the risk score associated with the patient based on the use of a certain treatment device. In this way, the control unit 20 may also determine a medical device related pressure injury risk. In such circumstances, the patient may or may not be part of the perioperative environment, but may be at the medical facility 40 for other treatment or care.

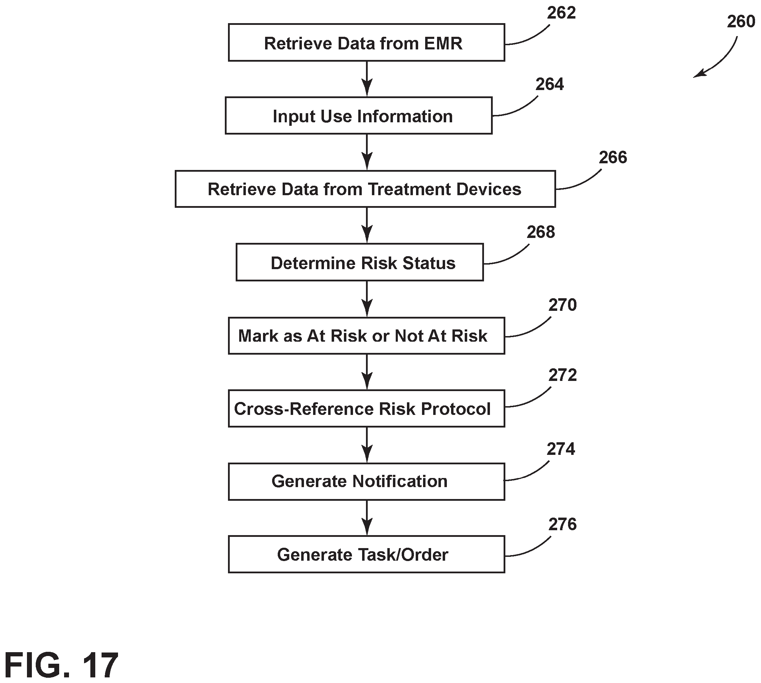

Referring to FIG. 17, as well as FIGS. 1-16, the control unit 20 may be configured to perform a method 260 of monitoring and tracking risk and/or development related to medical device related pressure injuries. In step 262, the control unit 20 collects data from the EMR 46 using a natural language process. The control unit 20 collects information relating to patient demographics, as well as information on treatment devices 56 used during a surgical procedure or otherwise used during treatment at the medical facility 40. For example, the data or information may include patient age, history of pressure injuries, a procedure surgery or treatment, a medical device (e.g., the treatment device 56) associated with the procedure, surgery, or treatment, comorbidities such as diabetes, patient weight, a perioperative pressure injury risk assessment such as the Braden score, the Norton score, the Waterlow score, or a combination thereof, the pressure injury risk assessment including the Scott Triggers, information transferred from another unit, whether the patient is coming from home, etc. The data collected and how the data is collected from the EMR 46 may be similar to the process described in step 202 of method 200 and step 232 of method 230.

In step 264, use information relating to various treatment devices 56 is input through the application interface 172 or otherwise input by the caregiver into the monitoring system 10. The manually input use information may relate to treatment devices 56 that may not include a controller in communication with the control unit 20. The use information may relate to the support apparatus 12, the vital signs monitor 64, the oxygen therapy device 66, the sequential compression device 130, the mattress 132, the SEM scanner 136, etc.

Additionally or alternatively, the use information may relate to any treatment devices 56, including, but not limited to the nasal cannula 74 used in association with the oxygen therapy device 66, oxygen tubing for ears, a Foley catheter, a cast, a splint, a cervical collar, a CPAP mask, a BiPAP mask, an endotracheal tube, a feeding tube, a halo brace, and a tracheostomy neck plate, other braces, positioning devices, therapy provided to the pneumatic system 134 of the mattress 132, the pressure within the mattress 132, offloading devices, anti-shear and friction pads, etc.

In step 266, the control unit 20 collects data from the treatment devices 56 in communication with the control unit 20 via the communication network 18. The treatment devices 56 may communicate data or the information in a similar manner as step 204 of method 200 and step 234 of method 230 according to any wireless or wired protocol as described herein.

In step 268, the control unit 20 determines the risk status of the patient for developing a medical device related pressure injury utilizing the data received in steps 262-266 of method 260. The control unit 20 may utilize rules-based algorithms in method 260. The control unit 20 may have a single rule that determines whether the patient is at risk. For example, if the patient has worn a neck brace for a period of time exceeding a predefined period of time, the patient is automatically marked as at risk. Additionally or alternatively, the control unit 20 may utilize multiple factors to determine whether the patient is at risk. For example, if the patient is over a certain age and a neck brace has been worn for a period of time greater than a predefined amount of time, then the patient is automatically marked as at risk.

In step 270, the control unit 20 marks the patient as at risk or not at risk in the patient profile 60. In step 272, the control unit 20 cross-references the risk status and the data received in steps 262-266 to the facility risk protocol 48 associated with the initial risk score, the risk status, the risk area 182, the pressure injury, or a combination thereof. The facility risk protocol 48 may be stored within the remote server 22. Accordingly, in step 272, the control unit 20 may communicate with the remote server 22 via the communication network 18 to cross-reference the risk with the facility risk protocol 48. The facility risk protocol 48 may guide the information to be included in the notification 24 and the treatment orders 188 to be generated by the control unit 20.

In step 274, the control unit 20 generates the notification 24 that is communicated to the caregiver via the application interface 172. The notification 24 generally indicates to the caregiver that the patient is at risk (e.g., the risk status), as well as includes contributing factors to the risk status, as illustrated in FIGS. 10 and 11. In step 276, the control unit 20 generates the treatment order 188 related to prevention treatment for the potential medical device related pressure injury. The notification 24 may include reminders, tasks, or treatment orders 188 associated with the risk status or at risk areas 182 of the patient (e.g., areas with greater or increased risk of a pressure injury), as illustrated in FIG. 12. The treatment orders 188 may include, for example, reminders to tape tubing, reminders to replace foam protectors, reminders to check for medical device related pressure injuries, etc. These notifications 24 may also appear in the EMR 46.

Additionally or alternatively, the treatment orders 188 may include the size and type of materials to use for the prevention treatment (e.g., the treatment order 188), as well as when to replace the materials. The control unit 20 may utilize information such as the location of the treatment device 56, the size of the patient, and the risk status of developing a medical device related pressure injury to provide treatment guidance to the caregiver. It is also contemplated that the notification 24 includes the facility risk protocols 48 retrieved from the remote server 22 to guide or prompt the treatment provided by the caregiver.

Additionally, in step 276, if the control unit 20 is in communication with the asset management system 98, the control unit 20 may send an alert or notification to the asset management system 98 to automatically order or prompt the ordering of materials that are low in stock or that a patient may need changed or utilized periodically. Whether the materials are low in stock may be stored in the remote server 22 or the local server 54. The materials the patient may need for various treatments and the frequency of changing the materials may be stored within the facility risk protocols 48 in the remote server 22. The treatment materials utilized by the caregiver may be communicated to the asset management system 98 to track the inventory of the treatment materials at the medical facility 40.

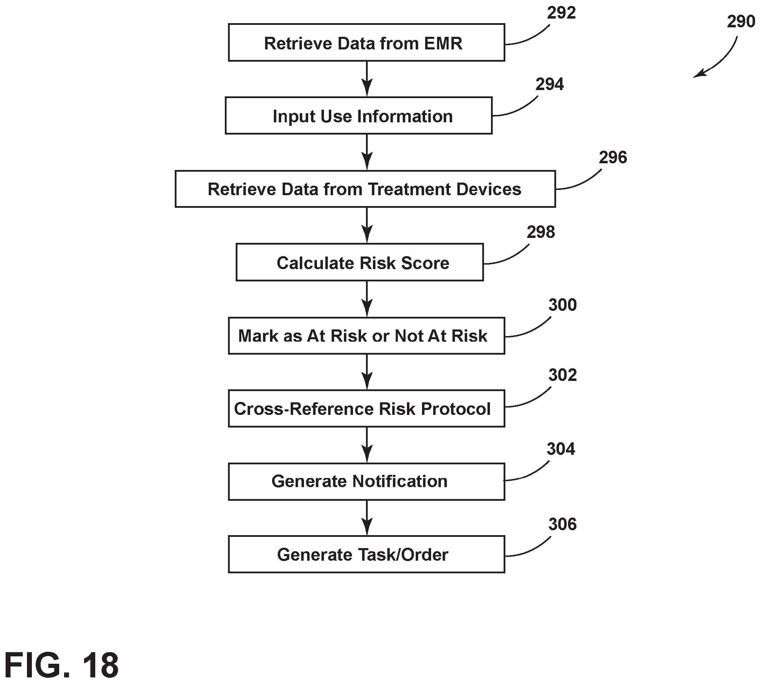

Referring to FIG. 18, as well as FIGS. 1-17, the control unit 20 may utilize a predictive algorithm for determining the likelihood of the patient to develop a medical device related pressure injury. The control unit 20 may perform method 290 to monitor and track the risk and/or development of the medical device related pressure injury. In step 292, the control unit 20 collects data from the EMR 46 with natural language processes, as described herein. The data or information may include patient age, history of pressure injuries, a procedure surgery or treatment, a medical device associated with the procedure, surgery, or treatment, comorbidities such as diabetes, patient weight, a perioperative pressure injury risk assessment such as the Braden score, the Norton score, the Waterlow score, or combination thereof, the pressure injury risk assessment including the Scott Triggers, information transferred from another unit, whether the patient is coming from home, etc.

In step 294, the caregiver inputs information regarding the use of certain treatment devices 56 that may or may not be in direct communication with the control unit 20. For example, information relating to the use of positioning devices or other device attachments in an intraoperative setting, the pulse oximetry sensor 68, the blood pressure cuffs 70, the nasal cannula 74, braces, off-loading devices, etc.

In step 296, the control unit 20 collects use data from the treatment devices 56 that are directly in communication with the control unit 20 via the communication network 18, according to any of the wired or wireless communication protocols described herein. The data is collected in a similar manner to step 266 of method 260 set forth herein. The use information of the treatment devices 56 may include the position of the patient on the support apparatus 12, time spent on the support apparatus 12, a specific surgical table 80, a specific position on the surgical table 80, positioning devices associated with the surgical table 80, etc.

In step 298, the control unit 20 may calculate the risk score of the patient utilizing the data received in steps 292-296 of method 290. The control unit 20 may store various weights associated with each factor or input assigned by a rules manager. The control unit 20 weighs the factors and determines the risk score of the patient for developing a medical device related pressure injury. Exemplary factors considered to calculate the risk score are set forth in Table 2 below.

| Table 2: Data Inputs | | | | | |
|---|---|---|---|---|---|
| EMR Data | | Intra-op Device Data | | Additional Device Data | |
| • | Patient age | • | Time on a surgical table | • | Position of the patient on bed |
| • | History of pressure injuries | • | Patient positions on a surgical table | | |
| • | Patient procedure or surgery | | | • | Continuous use of monitors, including blood pressure cuff, pulse oximeter, etc. |
| | | • | Cumulative time in each position | | |
| • | Comorbidities, such as diabetes, de-nutrition, vascular issues | | | | |
| | | • | Use of positioning devices | | |
| | | • | Micro-movements of patient | • | Specific operating room table |
| • | Smoking history | | | | |
| • | Patient weight, BMI | • | Use of warming devices | | |
| • | NPO status | • | Anesthetic medications and gases | | |
| • | Patient medication | | | | |
| • | PPI risk score from the perioperative environment | • | Medical device related pressure injury assessment | | |
| • | Lab data (Hbg/Hct, albumin, creatine, hydration-related, etc.) | | | | |
| | | • | Data from a surgical table on which device attachments were used | | |
| • | Data about transfer from another unit or from home | | | | |
| • | Use of medical devices (e.g., feeding tube, nasal oxygen tube) | | | | |

The control unit 20 may utilize the data received from the various inputs and calculate the risk score corresponding with the likelihood of developing the pressure injury. Additionally or alternatively, in step 298, the control unit 20 may combine the predictive algorithm with the rules-based algorithm as previously described herein. In such examples, the predictive algorithm weighing the various factors is utilized until a specific condition is recognized by the control unit 20. For example, the condition may be that a predetermined amount of time on the surgical table 80 or utilizing a certain treatment device 56 has exceeded a predefined period of time. Once the condition is recognized by the control unit 20, the rules-based algorithm may override the predictive algorithm.

In step 300, the control unit 20 determines whether the patient is at risk or not at risk of developing a medical device related pressure injury based on the calculated risk score. The control unit 20 may store a predefined value that is compared to the calculated risk score. If the risk score is above the predefined value, the control unit 20 marks the patient as at risk and indicates the severity of the risk. Additionally, if a certain condition has been met to override the predictive algorithm, the condition may be noted with the risk score.

In step 302, the control unit 20 cross-references the risk score, risk factors, at risk areas, etc. with the facility risk protocol 48 stored within the remote server 22. In step 304, the control unit 20 generates the notification 24 that is communicated to the caregiver. The notification 24 may include information relating to the risk score, the risk factors, as illustrated in FIGS. 10 and 11. In step 306, the control unit 20 generates the treatment orders 188 providing guidance on prevention treatment and associated materials to use for the prevention treatment, and the control unit 20 may update the asset management system 98, similar to step 276 of method 260.

It is contemplated that the steps of methods 200, 230, 260, and 290 may occur simultaneously or independently of one another. Additionally, the steps of the associated methods 200, 230, 260, 290 may be performed in any order, simultaneously, repeated, or omitted without departing from the teachings herein. Further, it is contemplated that each of the methods 200, 230, 260, 290 may not be separately applied methods, but various steps from each method may be conducted by the control unit 20 in any given order.

Use of the present system may provide for a variety of advantages. For example, the caregivers associated with the patient throughout the medical facility 40 may be in direct communication with one another, as well as caregivers at other medical facilities 40. Additionally, the history of the patient relating to incurring pressure injuries may be tracked through each stage of the treatment process and through each shift of caregivers in various units and departments. Also, any pressure injury obtained by the patient is communicated back to the caregivers. Further, the monitoring system 10 provides continuity of communication of ongoing pressure injury risk that follows the patient. The patient profile 60 may persist indefinitely to provide accurate treatment and care to the patient during each visit to any medical facility 40 connected to through the monitoring system 10. Moreover, the monitoring system 10 provides continued communication and assessment of the patient.

Additionally, the monitoring system 10 provides risk information and education to the patient or subsequent caregiver upon discharge (e.g., the discharge report 194). Also, the monitoring system 10 determines the risk status and/or calculates the risk score of the patient. Further, the monitoring system 10 generates notifications 24 to alert caregivers and provide treatment orders 188, tasks, or guidance on preventative materials to be used by the caregiver. Moreover, the monitoring system 10 may improve the care provided to the patient. Additionally, the monitoring system 10 provides the feedback report 196 to the caregivers associated with the patient if a pressure injury develops. The feedback report 196 may include the listing of risk factors that sets forth the factors that the monitoring system 10 utilized in determining the likely cause of the pressure injury. Also, the monitoring system 10 may be used for the perioperative environment, may be used to track medical device related injuries, or a combination thereof. The monitoring system 10 provides an interconnected communication system between each caregiver associated with the patient to improve the care provided to the patient. Additional benefits and advantages may be realized and/or achieved.

For purposes of this disclosure, the term "coupled" (in all of its forms, couple, coupling, coupled, etc.) generally means the joining of two components (electrical or mechanical) directly or indirectly to one another. Such joining may be stationary in nature or movable in nature. Such joining may be achieved with the two components (electrical or mechanical) and any additional intermediate members being integrally formed as a single unitary body with one another or with the two components. Such joining may be permanent in nature or may be removable or releasable in nature unless otherwise stated.

It is also important to note that the construction and arrangement of the elements of the disclosure, as shown in the exemplary embodiments, is illustrative only. Although only a few embodiments of the present innovations have been described in detail in this disclosure, those skilled in the art who review this disclosure will readily appreciate that many modifications are possible (e.g., variations in sizes, dimensions, structures, shapes, and proportions of the various elements, values of parameters, mounting arrangements, use of materials, colors, orientations, etc.) without materially departing from the novel teachings and advantages of the subject matter recited. For example, elements shown as integrally formed may be constructed of multiple parts or elements shown as multiple parts may be integrally formed, the operation of the interfaces may be reversed or otherwise varied, the length or width of the structures and/or members or connector or other elements of the system may be varied, the nature or number of adjustment positions provided between the elements may be varied. It should be noted that the elements and/or assemblies of the system may be constructed from any of a wide variety of materials that provide sufficient strength or durability, in any of a wide variety of colors, textures, and combinations.

Embodiments of the invention can be described with reference to the following numbered clauses, with additional features laid out in the dependent clauses:
1. A monitoring system for tracking pressure related injury risk, comprising: a support apparatus including: a controller, wherein the controller is configured to communicate data relating to the support apparatus; and communication circuitry operably coupled to the support apparatus, wherein the communication circuitry is configured to communicate via a communication network; and a control unit configured to communicate with at least one of a remote server and the support apparatus via the communication network, wherein the control unit is configured to: determine at least one of a risk status and a risk score for developing a pressure injury in response to communication from the at least one of the remote server and the support apparatus; determine at least one contributing risk factor for developing the pressure injury; and generate a notification configured to be communicated to a user interface assembly.
2. The monitoring system of clause 1, wherein the support apparatus is a surgical table.
3. The monitoring system of either of clause 1 or clause 2, wherein the control unit communicates with the user interface assembly to receive use information relating to a treatment device.
4. The monitoring system of any one of clauses 1-3, wherein the control unit communicates with the remote server to receive treatment materials associated with the pressure injury, and wherein the notification generated by the control unit includes the treatment materials.
5. A method of tracking pressure injuries, comprising: collecting data from an electronic medical record of a patient; collecting data from a support apparatus relating to use of the support apparatus; calculating a risk score of developing a pressure injury in response to the data received from at least one of the electronic medical record and the support apparatus; generating a notification including at least one of the risk score, a listing of risk factors, and areas of increased risk; and generating treatment orders based on at least one of the risk score, the listing of risk factors, and the areas of increased risk.
6. The method of clause 5, further comprising: generating a feedback report including at least one of details of pressure injury and contributing factors; and sending the feedback report to a remote device.
7. The method of either of clause 5 or clause 6, further comprising: generating a discharge report including at least one of a listing of risk factors, information on identifying pressure injuries, and information on when to contact a caregiver after identifying a pressure injury.
8. A method of monitoring medical device related pressure injuries, comprising: collecting first data from an electronic medical record; collecting second data from at least one of a treatment device, a support apparatus, and a user interface; calculating a risk score of developing a medical device related pressure injury with the first data and the second data; cross-referencing a protocol relating to the medical device related pressure injury; and generating a notification if the risk score indicates a patient is at risk for developing the medical device related pressure injury.
9. The method of clause 8, wherein the step of calculating the risk score includes at least one of identifying if a predefined condition is satisfied and comparing multiple predefined conditions.
10. The method of either of clause 8 or clause 9, further comprising: inputting use information relating to the treatment device utilized by a patient into the user interface.
11. The method of any one of clauses 8-10, further comprising: generating a task corresponding to at least one of prevention treatment for the medical device related pressure injury and materials to be used for the prevention treatment.
12. A monitoring system for tracking pressure related injury risk, comprising: a support apparatus having a controller, wherein the controller is configured to communicate data relating to the support apparatus; communication circuitry operably coupled to the support apparatus, wherein the communication circuitry is configured to communicate the data via a communication network; and a control unit configured to communicate with the support apparatus and a user interface assembly via the communication network, wherein the control unit is configured to: determine at least one of a risk status and a risk score for developing a pressure injury; determine an area of increased risk for developing the pressure injury; and generate a notification including a graphical representation of a person that is displayed via the user interface assembly.
13. The monitoring system of clause 12, wherein the notification includes an identifier on the graphical representation corresponding to the area of increased risk.
14. The monitoring system of clause 13, wherein the identifier includes a highlighted area on the graphical representation.
15. The monitoring system of any one of clauses 12-14, wherein the control unit is configured to communicate the notification to a remote server via the communication network for storage in an electronic medical record.
16. The monitoring system of any one of clauses 12-15, wherein the control unit is configured to generate an updated notification in response to communication from at least one of the support apparatus and the user interface assembly.
17. The monitoring system of any one of clauses 12-16, wherein the control unit is configured to determine whether a patient is at risk for developing a pressure injury based on a calculated risk score determined utilizing a predictive algorithm to weigh factors related to a patient and use of the support apparatus.
18. The monitoring system of any one of clauses 12-17, wherein the control unit is configured to determine whether a patient is at risk for developing a pressure injury based on satisfying a threshold factor utilizing a rules-based algorithm.
19. A monitoring system for tracking pressure related injury risk, comprising: a treatment device having a controller, wherein the controller is configured to communicate data relating to the treatment device; communication circuitry operably coupled to the treatment device, wherein the communication circuitry is configured to communicate the data via a communication network; and a control unit that communicates with at least one of the treatment device and a user interface assembly via the communication network, wherein the control unit is configured to: determine at least one of a risk status and a risk score for developing a pressure injury; generate a first notification configured to be communicated to the user interface assembly, wherein the first notification includes a selectable feature, and wherein the selectable feature corresponds to at least one of a risk status indicator, a risk score indicator, a risk assessment, a risk area, and a prevention treatment order; and generate a second notification in response to a selection of at least one of the selectable features, wherein the control unit is configured to communicate the second notification to the user interface assembly.
20. The monitoring system of clause 19, wherein the identifier is a highlighted area on the icon.
21. The monitoring system of either of clause 21 or clause 22, wherein the second notification includes selectable features corresponding to a discharge report and a feedback report.
22. The monitoring system of any one of clauses 21-23, wherein the control unit is configured to generate a third notification including at least one of the discharge report and the feedback report in response to a selection of one of the selectable feature of the second notification, wherein the control unit is configured to communicate the third notification to the user interface assembly.
23. The monitoring system of any one of clauses 21-24, wherein the control unit is configured to communicate at least one of the first notification and the second notification to a patient profile.
24. A monitoring system (10) for tracking pressure related injury risk, comprising:
   a support apparatus (12) including:
      a controller (14), wherein the controller (14) is configured to communicate data relating to the support apparatus (12); and
      communication circuitry (16) operably coupled to the support apparatus (12), wherein the communication circuitry (16) is configured to communicate via a communication network (18); and
   a control unit (20) configured to communicate with at least one of a remote server (22) and the support apparatus (12) via the communication network (18), wherein the control unit (20) is configured to:
      determine at least one of a risk status and a risk score for developing a pressure injury in response to communication with the at least one of the remote server (22) and the support apparatus (12);
      determine at least one contributing risk factor for developing the pressure injury; and
      generate a notification (24) configured to be communicated to a user interface assembly (26).
25. The monitoring system (10) of clause 24, wherein the control unit (20) is configured to communicate the notification (22) to a remote device (50), wherein the notification (24) includes a report (194, 196) that has at least one of the risk score, the risk status, and the at least contributing one risk factor related to at least one of the risk score and the risk status.
26. The monitoring system (10) of clause 25, wherein the report (194, 196) is a discharge report (194) including at least one of the at least one risk factor and data on contacting a caregiver for at least one of pressure injury related prevention and treatment.
27. The monitoring system (10) of clause 25, wherein the report (194, 196) is a feedback report (196) configured to be communicated to a caregiver, wherein the control unit (20) is configured to determine information related to a cause of a pressure injury and include the information in the feedback report (196).
28. The monitoring system (10) of any one of clauses 24-27, further comprising:
   a vital signs monitor (64) communicatively coupled with the control unit (20), wherein the vital signs monitor (64) is configured to communicate use information to the control unit (20), and wherein the use information includes time of use of the vital signs monitor (64).
29. The monitoring system (10) of any one of clauses 24-28, wherein the notification (24) includes a treatment order (186) including prevention treatment based on at least one of the risk status and the risk score.
30. The monitoring system (10) of clause 29, wherein the treatment order (186) includes treatment materials, and wherein the control unit (20) is configured to communicate the treatment materials utilized for the treatment order (186) to an asset management system (98) to monitor an inventory of the treatment materials.
31. The monitoring system (10) of any one of clauses 24-30, further comprising:
   a treatment device (56) in communication with the user interface assembly (26), wherein the user interface assembly (26) is configured to receive an input including use information about the treatment device (56) that is configured to be communicated to the control unit (20), and wherein the treatment device includes at least one of a vital signs monitor (64), a neck brace, a sequential compression device (130), oxygen tubing for ears, nasal oxygen tubing (74), a Foley catheter, a cast, a splint, a cervical collar, a continuous positive airway pressure mask, a bilevel positive airway pressure mask, an endotracheal tube, a feeding tube, a halo brace, and a tracheostomy neck plate.
32. The monitoring system (10) of any one of clauses 24-31, wherein the control unit (20) is configured to communicate with the remote server (22) to receive facility risk protocols (48) relating to at least one of the pressure injury and the risk score, and wherein the notification (24) generated by the control unit (20) includes at least one of the facility risk protocols (48) and a prompt for treatment based on the facility risk protocols (48).
33. The monitoring system (10) of any one of clauses 24-32, wherein the control unit (20) is configured to:
   determine an area of increased risk (182) for developing the pressure injury; and
   generate the notification (24) including a graphical representation (194) of a person that is displayed via the user interface assembly (26).
34. The monitoring system (10) of clause 33, wherein the notification (24) includes an identifier (186) on the graphical representation (184) corresponding to the area of increased risk (182).
35. The monitoring system (10) of any one of clauses 24-34, wherein the control unit (20) is configured to generate an updated notification (24) in response to communication from at least one of the support apparatus (12) and the user interface assembly (26).
36. The monitoring system (10) of clause 35, wherein the notification (24) includes a prevention treatment order (186) including a listing of tasks, and wherein the updated notification (24) includes completion identifiers (190) corresponding to completed tasks.
37. The monitoring system (10) of any one of clauses 24-36, wherein the control unit (20) is configured to determine whether a patient is at risk for developing a pressure injury based on a calculated risk score determined utilizing a predictive algorithm to weigh factors related to the patient and use of the support apparatus (12).
38. The monitoring system (10) of any one of clauses 24-37, wherein the control unit (20) is configured to determine whether a patient is at risk for developing a pressure injury based on satisfying a threshold factor utilizing a rules-based algorithm.

## Claims

1. A method of tracking pressure injuries, comprising:
collecting data from an electronic medical record of a patient;
collecting data from a support apparatus relating to use of the support apparatus;
calculating a risk score of developing a pressure injury in response to the data received from at least one of the electronic medical record and the support apparatus;
generating a notification including at least one of the risk score, a listing of risk factors, and areas of increased risk; and
generating treatment orders based on at least one of the risk score, the listing of risk factors, and the areas of increased risk.

2. The method of claim 1, further comprising:
generating a feedback report including at least one of details of pressure injury and contributing factors; and
sending the feedback report to a remote device.

3. The method of either one of claims 1 or 2, further comprising: g
generating a discharge report including at least one of a listing of risk factors, information on identifying pressure injuries, and information on when to contact a caregiver after identifying a pressure injury.

4. The method of any one of claims 1-3, wherein collecting data from the support apparatus includes collecting the data from at least one of a medical bed and a surgical table.

5. The method of claim 4, collecting the data from the support apparatus includes collecting the data from sensors configured to monitor at least one of position of a patient, movement of the patient, and physiological data of the patient.

6. The method of either one of claims 4 or 5, further comprising:
collecting data from an additional treatment device including at least one of a mattress and a therapeutic surface including a pneumatic system.

7. The method of claim 6, further comprising:
generating a task corresponding to at least one of prevention treatment and materials to be used for the prevention treatment.

8. The method of claim 7, wherein generating the task includes generating treatment orders that include at least one of a change in the therapeutic surface and at least one of therapy provided by the pneumatic system

9. The method of any one of claims 1-8, further comprising:
determining a likelihood of a pressure injury developing; and
determining a cause of a pressure injury that has developed.

10. The method of claim 9, wherein determining the likelihood and the cause includes:
receiving and utilizing at least one of a time of the patient on the support apparatus, a time of the patient in a position on the support apparatus, nutrition, moisture, measured micro-climate, use of monitors, and use of devices.

11. The method of claim 9, wherein determining the likelihood and the cause includes:
receiving and utilizing at least one of a type of procedure, a length of procedure, a device used with a procedure, nutrition, moisture, and history of pressure injuries from the electronic medical record.

12. The method of any one of claims 1-11, further comprising:
determining whether a patient is at risk for developing a pressure injury based on a calculated risk score determined utilizing a predictive algorithm to weigh factors related to the patient and use of the support apparatus.

13. The method of any one of claims 1-12, further comprising:
determining whether a patient is at risk for developing a pressure injury based on satisfying a threshold factor utilizing a rules-based algorithm.

14. The method of any one of claims 1-13, wherein calculating the risk score includes at least one of identifying if a predefined condition is satisfied and comparing multiple predefined conditions

15. The method of any one of claims 1-14, further comprising:
receiving facility risk protocols relating to at least one of the pressure injury and the risk score; and
generating a notification including at least one of the facility risk protocols and a prompt for treatment based on the facility risk protocols.
